**Europäisches Patentamt**

**European Patent Office**

**Office européen des brevets**

(11) Veröffentlichungsnummer: **0 092 708**
**A2**

## EUROPÄISCHE PATENTANMELDUNG

(21) Anmeldenummer: 83103384.0

(22) Anmeldetag: 07.04.83

(51) Int. Cl.³: **A 61 K 9/24**
**A 01 N 25/26, C 05 G 3/00**

(30) Priorität: 21.04.82 DE 3214667

(43) Veröffentlichungstag der Anmeldung:
02.11.83 Patentblatt 83/44

(84) Benannte Vertragsstaaten:
AT BE CH FR GB LI NL

(71) Anmelder: Akzo GmbH
Postfach 10 01 49 Kasinostrasse 19-23
D-5600 Wuppertal-1(DE)

(72) Erfinder: Lange, Wolfgang, Dr. Dipl.-Chem.
Rosenstrasse 12
D-8753 Obernburg(DE)

(72) Erfinder: Boer, G.J., Dr.
Frans van Mierislaan 5
NL-1399 GD Muiderberg(NL)

(54) Zusammengesetzter Körper für die Langzeitabgabe von Wirkstoffen.

(57) Der zusammengesetzte Körper dient zur Langzeitabgabe von Wirkstoffen und besteht aus einem mikroporösen Polymer als Trägersubstanz und einem Überzug aus Cellulosenitrat. Die Trägersubstanz besteht aus einem thermoplastischen Polymer mit einer im wesentlichen isotropen mikroporösen cellförmigen Polymerstruktur, die aus einer Vielzahl von im wesentlichen sphärischen Zellen eines mittleren Durchmessers von etwa 0,5 bis 100 µm besteht. Die Zellen sind untereinander durch Poren verbunden. Der zusammengesetzte Körper dient vor allem zur Langzeitabgabe von Peptiden, Hormonen, Neurotransmittern, insbesondere von Vasopressin und Oxytocin.

EP 0 092 708 A2

Croydon Printing Company Ltd.

**0092708**

A3GW32015 EP

Zusammengesetzter Körper für die
Langzeitabgabe von Wirkstoffen

A k z o    GmbH

Wuppertal

Die Erfindung betrifft zusammengesetzte Körper für die Langzeitabgabe von Wirkstoffen wie mehrschichtige Membranen,
mehrschichtige Hohlfasern oder mit einer Haut oder einer Membranschicht überzogene poröse Pulver u.dgl., die in der Lage
sind, Wirkstoffe wie z.B. Pharmazeutika, Hormone, Chemikalien
u.dgl. über lange Zeit in gleichen Mengen an ihre Umgebung
abzugeben.

Von der pharmazeutischen Industrie werden immer neue und
bessere Wirkstoffe entwickelt. Im Gegensatz dazu haben sich
die Methoden der Applikation solcher Wirkstoffe kaum verändert,
denn noch immer werden die meisten Medikamente oral oder
selterner subkutan, intramuskulär oder intravenös appliziert.
Dabei ist die Dosierung eines Wirkstoffs für eine erfolgreiche Therapie oft von ebenso großer Bedeutung wie der Wirkstoff selbst.

Man hat sich bemüht, die Nachteile der herkömmlichen Applikation dadurch zu vermeiden, daß man den Wirkstoff aus einem Depot mit einer bestimmten Rate abgibt. So gibt es z.B. sogenannte Mikrokapseln, bei denen der Wirkstoff, im allgemeinen ein Arzneimittel, von einer Membran umhüllt ist. Es ist dabei möglich, die Austrittsgeschwindigkeit des Arzneimittels zu steuern, z.B. durch die Struktur der Membran, deren Dicke, durch Auswahl des Polymers für die Membran usw. Man hat auch schon mikroporöse Pulver mit Wirkstoffen wie Düngemittel, Schädlingsbekämpfungsmittel etc. beladen, welche dann im Laufe der Zeit die Wirkstoffe an ihre Umgebung abgeben.

Bei all diesen Verfahren läßt es sich vielfach nicht vermeiden, daß zumindest am Anfang eine erhöhte Abgabe des Wirkstoffs stattfindet, man spricht hier von einem sogenannten "burst effect". Häufig nimmt im Laufe der Zeit die Abgabemenge noch ab, obwohl das Depot noch lange nicht erschöpft ist.

Es ist auch bekannt, bei derartigen Vorgängen zusammengesetzte Membranen einzusetzen oder poröse Körnchen, die einen Wirkstoff enthalten, mit einem Imprägniermittel zu versehen. So wird z.B. in der europäischen Patentschrift 5302 ein Verfahren beschrieben, bei dem Körnchen mit einem Wirkstoff beladen werden und man durch chemische Reaktion eine Polyurethanhaut bildet, welche die Poren abschließen soll. Ein solches Verfahren ist kompliziert und umständlich.

Obwohl bereits eine ganze Reihe von Körpern für die Langzeitabgabe von Wirkstoffen bekannt sind, besteht noch ein Bedürfnis nach verbesserten Körpern für die Langzeitabgabe.

Aufgabe der Erfindung ist es deshalb, einen zusammengesetzten Körper zur Verfügung zu stellen, der sich auf einfache wirtschaftliche Weise herstellen läßt und der als Träger von Wirkstoffen in der Lage ist, die Wirkstoffe gleichmäßig über einen längeren Zeitraum an die Umgebung abzugeben. Aufgabe der Erfindung ist es ferner, einen zusammengesetzten Körper zur Verfügung zu stellen, der nicht so ohne weiteres durch Einflüsse von der äußeren Umgebung, z.B. durch Verstopfung oder Ausbildung einer Haut blockiert wird, so daß die Abgabe des Wirkstoffes reduziert oder sogar gestoppt wird.

Aufgabe der Erfindung ist es ferner, einen zusammengesetzten Körper für die Langzeitabgabe zur Verfügung zu stellen, der für eine Reihe von Substanzen, welche sich in der äußeren Umgebung befinden und den Wirkstoff desaktivieren könnten, nicht durchlässig ist.

Diese Aufgabe wird durch einen zusammengesetzten Körper für die Langzeitabgabe von Wirkstoffen gelöst, der gekennzeichnet ist, durch ein mikroporöses Polymer als Trägersubstanz für den Wirkstoff und einen Überzug als Cellulosenitrat. Vorzugsweise dient als Trägersubstanz ein thermoplastisches Polymer mit einer im wesentlichen isotropen mikroporösen zellförmigen Polymerstruktur, die aus einer Vielzahl von im wesentlichen sphärischen Zellen eines mittleren Durchmessers von etwa 0,5 bis 100 µm und die angrenzenden Zellen untereinander verbindenden Poren besteht, wobei der Durchmesser der Poren kleiner ist als der der sphärischen Zellen und das Verhältnis des durchschnittlichen Zellendurchmessers zum durchschnittlichen Porendurchmesser etwa 2 : 1 bis etwa 200 : 1 beträgt.

Vorzugsweise ist der mittlere Durchmesser der Zellen etwa 0,5 bis 10 µm und der mittlere Durchmesser der Poren etwa 0,1 bis 1 µm. Sehr geeignet als Träger ist ein mikroporöses Polymer in Form einer Hohlfaser. Die Hohlfaser weist vorzugsweise eine glatte Öffnungen aufweisende Oberfläche auf, wobei der Anteil der Öffnungen in der Fläche 10 bis 90 % beträgt.

Der mikroporöse Träger kann auch in Form von Pulver vorliegen.
Als Polymer ist für die zusammengesetzten Körper insbesondere
Polypropylen sehr geeignet. Der Hohlraumanteil des Trägers,
der im wesentlichen aus dem Volumen der sphärischen Zellen
und der Poren besteht, kann zwischen etwa 10 und 90 % betragen.
Ein Hohlraumanteil von etwa 70 bis 80 % ist besonders vorteilhaft. Die zusammengesetzten Körper gemäß der Erfindung eignen
sich insbesondere zur Abgabe von Peptiden, Hormonen, Neurotransmittern, Vasopressin und Oxytocin.

Die Herstellung von Trägern mit einer Struktur, wie sie
gemäß der Erfindung erforderlich ist, wird in der DE-OS
27 37 745 beschrieben, auf deren Offenbarung sich hier ausdrücklich bezogen wird. Dort werden zahlreiche Polymere und spezielle
organische Flüssigkeiten erwähnt, die sich nach dem dort angegebenen Verfahren zu den verschiedensten Formkörpern mit entsprechender mikroporöser Struktur verarbeiten lassen. Als
Polymere können grundsätzlich alle plastischen Polymere verwendet werden; besonders geeignet sind Polyolefine wie Polypropylen und Polyäthylen, auch Polyamid ist geeignet.

Diese geformten Träger können bereits bei ihrer Herstellung
mit dem Wirkstoff beladen werden oder nach einer in der zitierten DE-OS 27 37 745 offenbarten Methode nachträglich mit Wirkstoff gefüllt werden.

Hohlfasermembranen, wie sie im Rahmen der Erfindung vorzugsweise als Träger verwendet werden, können nach einem Verfahren
hergestellt werden, das in der DE-OS 28 33 493 offenbart wird.
Auf die Lehre dieser DE-OS wird sich im Rahmen dieser Erfindung
insbesondere bezogen.

Mikroporöse Pulver, welche als Träger im Rahmen der Erfindung
geeignet sind, werden insbesondere beschrieben in der DE-OS
30 26 762 und 30 26 688.

Die Offenbarung der vorstehend genannten Offenlegungsschriften soll ausdrücklich in den Rahmen der vorliegenden
Anmeldung miteinbezogen werden.

Die Träger sind im Rahmen der Erfindung mit einem Überzug
aus Cellulosenitrat versehen. Cellulosenitrat ist ein anorganischer Ester der Cellulose, der in den verschiedensten
Veresterungsgraden erhältlich ist. Als Maß für den Veresterungsgrad wird häufig der Stickstoffgehalt angegeben.
So kann der Stickstoffgehalt z.B. Werte von 10,5 oder 12,9
besitzen. Auch höhere Werte bis zu 14,14 % N der theoretischen Menge für das Trinitrat, sind möglich.

Als Cellulosenitrat gemäß der Erfindung läßt sich insbesondere
auch sehr gut Collodium verwenden. Nähere Angaben über Cellulosenitrat und Collodium sind z.B. in RÖMPPS CHEMIE-LEXIKON
Franckh'sche Verlagshandlung Stuttgart (7. Auflage) auf den
Seiten 530-531 und 676 zu finden.

Die Ausbildung des Überzugs aus Cellulosenitrat bzw. Collodium
auf dem Träger kann auf verschiedene Weise geschehen. So
ist es z.B. möglich, eine Lösung von Collodium auf den Träger
aufzusprühen, wobei sich nach Entfernen des Lösungsmittels
eine entsprechende Haut aus Collodium bildet.

In vielen Fällen kann man auch den Träger durch ein- oder vorzugsweise mehrfaches Eintauchen in eine Collodiumlösung und
Einschalten von Zwischentrocknungen auf geeignete Weise mit
dem Überzug versehen. Durch die Höhe der Konzentration der
Collodiumlösung und die Häufigkeit des Eintauchens läßt sich
die Dicke des Überzugs steuern. Für viele Verwendungszwecke
liegen brauchbare Dicken des Überzugs in der Größenordnung von
10 oder 100 µm.

Der zusammengesetzte Körper gemäß der Erfindung ist für die verschiedensten Zwecke, bei denen es auf die Langzeitabgabe von Wirkstoffen ankommt, einsetzbar. So lassen sich z.B.. in der Land- und Fortwirtschaft vor allem zusammengesetzte Körper in Pulver- oder Granulatform verwenden, wenn sie mit Wirkstoffen wie Insektiziden, Herbiziden oder auch Düngemitteln, Spurenelementen u.dgl. beladen sind.

Eine besonders vorteilhafte Verwendungsmöglichkeit bieten die zusammengesetzten Körper gemäß der Erfindung bei der Applikation von pharmazeutisch wirksamen Substanzen, Hormonen u.dgl., die in den menschlichen oder tierischen Körper eingebracht werden sollen. So kann man z.B. eine Hohlfaser, die an einem Ende zugeschweißt ist, wie eine Sonde in den menschlichen oder tierischen Körper einbringen, das Innere der Sonde mit einem Wirkstoff, z.B. einem Hormon füllen, das dann langsam und gleichmäßig an den Körper abgegeben wird.

Für sehr lange Behandlungszeiten ist es möglich, die Sonde nach einer bestimmten Zeit von außen wieder zu füllen. Man kann die Sonde auch an ein Vorratsgefäß anschließen.

Besonders vorteilhaft lassen sich zusammengesetzte Körper gemäß der Erfindung bei der Behandlung in der Tier- und Humanmedizin verwenden. Ein bevorzugtes Einsatzgebiet ist die Behandlung mit Hormonen, Neurotransmittern, insbesondere Vasopressin und Oxytocin.

So kann man z.B. einen hohlfaserförmigen Träger, z.B. aus Polypropylen oder auch einen dünnen Schlauch aus dem gleichen oder einem ähnlichen Material innen mit einer Vasopressin- Lösung füllen, die Hohlfaser bzw. den dünnen Schlauch an den beiden Enden z.B. durch Verschweißen schließen und mit einem Collodiumüberzug versehen.

Ein solcher Schlauch bzw. eine solche Hohlfaser ist aufgrund seiner Flexibilität und seiner geringen Ausmaße leicht ganz oder teilweise in den menschlichen oder tierischen Körper einzuführen oder zu implantieren. So kann man z.B. eine Hohlfaser als Kanüle direkt an bestimmte Organe, z.B. in das Gehirn einführen. Auf diese Weise ist es möglich, lokale oder organspezifische Applikationen von Hormonen durchzuführen.

Mit Hilfe des zusammengesetzten Körpers gemäß der Erfindung war es möglich, an Ratten eine 4-wöchige Behandlung mit Vasopressin durchzuführen, während derer eine gleichmäßige konstante Abgabe des Wirkstoffs erfolgte, so daß das Vasopressin in günstiger Weise u.a. eine antidiuretische Wirkung entfalten konnte.

Ein besonderer Vorteil der erfindungsgemäßen Körper ist, daß sie für viele Stoffe, welche sich in der Umgebung befinden, nicht durchlässig sind. So können z.B. bei der Behandlung mit Hormonen bestimmte Proteine nicht in das Innere des Trägers eindringen und den Wirkstoff desaktivieren. So ist gewährleistet, daß über lange Zeit hinaus der zusammengesetzte Körper seine Wirksamkeit nicht verliert.

Es ist möglich, die Geschwindigkeit der Abgabe durch die Dicke des Trägerkörpers und des Überzugs aus Cellulosenitrat zu steuern. In vielen Fällen genügt ist, wenn die Haut aus Cellulosenitrat eine Dicke in der Größenordnung von 10 oder 100 µm aufweist. Eine weitere Möglichkeit der Applikation ist, daß man einen entsprechend beladenen zusammengesetzten Körper unter die Haut implantiert und nach einer bestimmten Behandlungsdauer wieder entfernt.

Vasopressin und Oxytocin, welche gemäß der Erfindung vorzugsweise verwendet werden, sind Hormone des Hypophysenhinterlappens. Vasopressin steuert u.a. die Nierentätigkeit; es ist ferner wirksam, als Neurotransmitter. Neurotransmitter sorgen im Nervensystem für die Signalweiterleitung.

Vasopressin kann ferner die Gedächnisleistung verbessern.

Es war besonders überraschend, daß sich mit den zusammengesetzten Körpern gemäß der Erfindung eine hervorragende gleichmäßige Wirkstoffabgabe über lange Zeit gewährleisten läßt. Insbesondere ist es mit den erfindungsgemäßen Körpern möglich, die anfänglich erhöhte Abgabe des Wirkstoffs, welche bei den bisher bekannten Mikrokapseln häufig zu beobachten ist, zu vermeiden, so daß es nicht beim Anfang einer Behandlung zu einem oft gefährlichen Wirkstoffstoß kommt.

Die zusammengesetzten Körper gemäß der Erfindung sind sehr vielseitig verwendbar. So können sie auch auf landwirtschaftlichen und forstwirtschaftlichen Gebieten eingesetzt werden, sei es zur Abgabe von Düngemitteln, Spurenelementen, Schädlingsbekämpfungsmitteln u.dgl. So können sie Träger von Herbiziden, Pestiziden, Duftstoffen, Sexuallockstoffen u.dgl. sein.

Die Herstellung der erfindungsgemäßen zusammengesetzten Körper ist äußerst einfach und wirtschaftlich durchzuführen.

Patentansprüche
_____

1.  Zusammengesetzter Körper für die Langzeitabgabe von
    Wirkstoffen, gekennzeichnet durch ein mikroporöses
    Polymer als Trägersubstanz und einen Überzug aus
    Cellulosenitrat.

2.  Zusammengesetzter Körper nach Anspruch 1, gekennzeichnet
    durch ein thermoplastisches Polymer mit einer im wesentlichen isotropen mikroporösen zellförmigen Polymerstruktur,
    die aus einer Vielzahl von im wesentlichen sphärischen
    Zellen eines mittleren Durchmessers von etwa 0,5 bis 100 µm
    und die angrenzenden Zellen untereinander verbindenden
    Poren besteht, wobei der Durchmesser der Poren kleiner
    ist als der der sphärischen Zellen und das Verhältnis des
    durchschnittlichen Zellendurchmessers zum durchschnittlichen Porendurchmesser etwa 2 : 1 bis etwa 200 : 1
    beträgt.

3.  Zusammengesetzter Körper nach Anspruch 2, gekennzeichnet
    durch sphärische Zellen eines mittleren Durchmessers von
    etwa 0,5 bis 10 µm und Poren eines mittleren Durchmessers
    von etwa 0,1 bis 1 µm.

4.  Zusammengesetzter Körper nach den Ansprüchen 1 bis 3,
    gekennzeichnet durch einen Träger in Form einer Hohlfaser.

5.  Zusammengesetzter Körper nach Anspruch 4, gekennzeichnet
    durch eine Hohlfaser mit einer glatten, Öffnungen aufweisen-
    den Oberfläche, wobei der Anteil der Öffnungen in der
    Fläche 10 bis 90 % beträgt.

6. Zusammengesetzter Körper nach den Ansprüchen 1 bis 3, gekennzeichnet durch einen pulverförmigen Träger.

7. Zusammengesetzter Körper nach den Ansprüchen 1 bis 6, gekennzeichnet durch Polypropylen als Trägersubstanz.

8. Mikroporöser Körper nach den Ansprüchen 1 bis 7, gekennzeichnet durch einen Hohlraumanteil des mikroporösen Trägers von 10 bis 90 %.

9. Zusammengesetzter Körper nach Anspruch 8, gekennzeichnet durch einen Hohlraumanteil von 70 bis 80 %.

10. Verwendung der zusammengesetzten Körper nach den Ansprüchen 1 bis 9, zur Abgabe von Peptiden.

11. Verwendung der zusammengesetzten Körper nach den Ansprüchen 1 bis 9, zur Abgabe von Hormonen.

12. Verwendung der zusammengesetzten Körper nach den Ansprüchen 1 bis 9, zur Abgabe von Neurotransmittern.

13. Verwendung der zusammengesetzten Körper nach den Ansprüchen 1 bis 9, zur Abgabe von Vasopressin.

14. Verwendung der zusammengesetzten Körper nach den Ansprüchen 1 bis 9, zur Abgabe von Oxytocin.